Europäisches Patentamt

European Patent Office

Office européen des brevets

(11) Veröffentlichungsnummer: **0 652 288 A1**

## (12) EUROPÄISCHE PATENTANMELDUNG

(21) Anmeldenummer: **94117180.3**

(22) Anmeldetag: **31.10.94**

(51) Int. Cl.6: **C12P 1/00**, C12N 1/00, C12M 1/36, C12N 9/80, C12N 15/55, //C12P1:00, C12R1:19

(30) Priorität: **05.11.93 DE 4337787**

(43) Veröffentlichungstag der Anmeldung: **10.05.95 Patentblatt 95/19**

(84) Benannte Vertragsstaaten: **BE DE DK ES FR GB GR IT LU NL PT**

(71) Anmelder: **HOECHST AKTIENGESELLSCHAFT Brüningstrasse 50**

D-65929 Frankfurt am Main (DE)

(72) Erfinder: **Aretz, Werner, Dr. Am Wäldchen 13 D-61462 Königstein (DE)**
Erfinder: **Willems, Philipp Idsteiner Weg 13 D-62559 Waldems (DE)**

(54) **Sauerstoffabhängige Fermentation von Mikroorganismen.**

(57) Sauerstoffabhängige Mikroorganismen, lassen sich vorteilhaft fermentieren, wenn man die Kohlenstoff-Fütterung in Abhängigkeit von der Sauerstofftransferrate vornimmt.

Standard 28 °C

Standard 25 °C

Direktbeimpfung 28 °C

Fig. 1

Rank Xerox (UK) Business Services
(3.10/3.09/3.3.4)

EP 0 652 288 A1

Die vorliegende Erfindung betrifft eine wachstumsgekoppelte Fedbatch-Fermentation von sauerstoffabhängigen Mikroorganismen.

Um gute Biomasse- bzw. Produktausbeute bei der Fermentation von Mikroorganismen zu erhalten, ist es bisher nötig, eine empirisch ermittelte optimale Fermentationsweise anzuwenden, wobei die Fermentationsparameter bei Veränderung der Randbedingungen (Animpfungsmethode, Zusammensetzung der Fermentationsbrühe, Temperatur etc.) nach der Methode "trial and error" jeweils neu angepaßt werden müssen und häufig nicht die optimale Fermentationsweise erreicht wird.

Überraschenderweise wurde nun eine Fermentationsweise von sauerstoffabhängigen Mikroorganismen gefunden, die unabhängig von verschiedenen Fermentationsparametern zu optimalen Ergebnissen führt, wenn die Kohlenstoff-Fütterung des Mikroorganismus in Abhängigkeit von der Sauerstofftransferrate erfolgt. Dies ist insbesondere überraschend, weil die Kohlenstoff-Fütterung bei sauerstoffabhängigen Mikroorganismen problematisch ist. So führt z.B. eine Überfütterung des Mikroorganismus mit Glycerin zu einer Akkumulation desselben, was die Bildung von Acetat, Wachstumsstillstand und den Abbruch der Produktbildung zur Folge hat.

Erfindungsgegenstand ist demzufolge eine Fermentation von sauerstoffabhängigen Mikroorganismen, die dadurch gekennzeichnet ist, daß die Kohlenstoff-Fütterung des Mikroorganismus in der Fermentationsbrühe in Abhängigkeit von der Sauerstofftransferrate (OUR) erfolgt.

Die erfindungsgemäße Fermentation kann zu unterschiedlichen Zwecken eingesetzt werden, besondere Bedeutung besitzt sie bei der Produktion von Proteinen, insbesondere von rekombinanten Proteinen.

Gemäß der vorliegenden Erfindung lassen sich unterschiedliche Mikroorganismen fermentieren, wie z.B. Mikroorganismen der Gattung Escherichia, Streptomyces, Bacillus oder Pseudomonas.

Besonders geeignet für die erfindungsgemäße Fermentation sind Bakterien der Gattung Escherichia. Ganz besonders geeignet ist die Art Escherichia coli. Von dieser besitzen besondere Bedeutung die Stämme W 3110 M (ATCC 27325), DH 1 (ATCC 33849), K 12, B, HB 101, JM 105, TG 1, MRC 1, RH 1, IH 1776. Ganz besondere Bedeutung besitzen die Stämme W 3110 M und DH 1. Die besondere Bedeutung der obengenannten Stämme resultiert unter anderem daraus, daß sie (als Rezipienten Stämme) nach gentechnischer Veränderung mit dem Ziel bestimmte Stoffe zu exprimieren optimal fermentiert werden können. Beispielsweise eignet sich das Verfahren zur Herstellung von Proteinen wie Glutarylacylase, Insulin, Interferonen, Hirudin, Erythropoietin etc..

Insbesondere besitzt das erfindungsgemäße Verfahren Bedeutung bei der Produktion von Glutarylacylase, z.B. indem das Plasmid pCM 145 (DSM 6409) (vgl. EP 0 504 798 A1, auf die ausdrücklich Bezug genommen wird) in einem Mikroorganismus (z.B. E. coli, insbesondere W 3110 M, DH 1) zur Expression gebracht wird.

Als Kohlenstoff-Fütterungsmedien kommen unterschiedliche Stoffe in Frage, ein besonders geeignetes Substrat ist Glycerin. Die übrigen Nährmittel entsprechen in Zusammensetzung und Konzentration denen üblicher Fermentationshilfsmittel; besonders geeignete Zusammensetzungen sind in den Beispielen wiedergegeben (vgl. auch z.B. Molecular Cloning, Sambrook, Fritsch, Maniatis, Cold Spring Laboratory Press, 1989).

Die Fermentation gemäß der vorliegenden Erfindung kann bei unterschiedlichen Temperaturen durchgeführt werden. Im Falle von Escherichia coli liegt die bevorzugte Temperatur bei ca. 28°C. Ein besonderer Vorteil der vorliegenden Erfindung ist jedoch, daß die Fermentation auch bei höherer oder niedrigerer (z.B. 22 bis 37°C) Temperatur durchgeführt werden kann, ohne daß alle Fermentationsparameter angepaßt werden müssen. Die Kohlenstoff-Fütterung wird quasi automatisch angepaßt über die Detektion der Sauerstofftransferrate und die Ermittlung der betreffenden Grenzwerte.

Die Kohlenstoff-Fütterung erfolgt bei der erfindungsgemäßen Fermentationsweise in der Weise, daß die Fütterung anfänglich proportional zur Sauerstofftransferrate erfolgt, bis sie einen empirisch zu ermittelnden Grenzwert erreicht. Anschließend erfolgt eine konstante Zufütterung mit der Kohlenstoffquelle, bis die Fermentation abgeschlossen ist; die Höhe der konstanten Zufütterung muß ebenfalls empirisch ermittelt werden. In der Regel beginnt nach Beginn der konstanten Zufütterung bei gentechnisch veränderten Rezipientenstämmen die Hauptproduktionsphase.

Es ist besonders vorteilhaft, die beschriebene Zufütterung computergesteuert vorzunehmen. Ein besonders geeigneter Algorithmus liest sich wie folgt:

Grundformel:       $SF = (Flag1 < 1)*(OUR_{act}/K) + (Flag1*K_{prod})$

Hilfsvariable 1:      $Flag1 = [(Flag1 > 0) + (OUR_{act} > OUR_{opt}) + ((OUR_{act} < OUR_{min})*Flag2)] > 0$

Hilfsvariable 2:      $Flag2 = [(Flag2 > 0) + (OUR_{act} > (OUR_{min} + 5))] > 0$

Der genannte Algorithmus gilt universell; die in Klammern nachfolgend angegebenen Werte gelten beispielhaft insbesondere für den Mikroorganismus W 3110 M. Die genannten Abkürzungen haben die folgende Bedeutung:

2

SF = Pumprate (ml/l*h)

Flag1 = Schalter, der die OUR-abhängige Pumprate der ersten Wachstumsphase abschaltet und die konstante Pumprate der Produktionsphase anschaltet (logische Abfrage mit dem Ergebnis 1 = richtig oder 0 = falsch; zu Fermentationsbeginn ist dieser Schalter auf 0 gesetzt; sobald $OUR_{act}$ größer $OUR_{opt}$ oder Flag2 gesetzt ist und $OUR_{act}$ kleiner $OUR_{min}$ ist, wird Flag 1 gesetzt)

Flag2 = Schalter, der zu Beginn der Fermentation auf 0 gesetzt ist; sobald $OUR_{act}$ größer ($OUR_{min}$ + 5) ist, wird Flag2 gesetzt und bleibt bis zum Schluß gesetzt

K = Pumpkonstante = $OUR_{max}/K_{prod}$ (hier als Beispiel 17,5)

$OUR_{act}$ = wachstumsabhängige, aktuelle Sauerstofftransferrate

$OUR_{max}$ = Grenzwert der Sauerstofftransferrate (vorzugsweise 60 bis 200, besonders bevorzugt 80 bis 160, insbesondere 100 bis 140 mMol/l*h; hier als Beispiel 120 mMol/l*h)

$OUR_{opt}$ = temperaturabhängige optimale Sauerstofftransferrate (vorzugsweise 60 bis 200, besonders bevorzugt 80 bis 160, insbesondere 90 bis 120 mM/l*h; hier als Beispiel 120 mMol/l*h bei Fermentationstemperatur von 28°C, 90 mMol/l*h bei Fermentationstemperatur von 25°C)

$OUR_{min}$ = minimale Sauerstofftransferrate (s. Flag2) (50 bis 90 mMol/l*h, hier als Beispiel 70 mMol/l*h bei Fermentationstemperatur 28°C)

$K_{prod}$ = Pumprate während Produktionsphase (6,85 ml/l*h) vorzugsweise 4 bis 9, besonders bevorzugt 5 bis 8, insbesondere 6,5 bis 7,5 ml/l*h; hier als Beispiel 6.85 ml/l*h)

Mit dem beschriebenen Algorithmus ist unabhängig von der Qualität des Inokulums und der Fermentationstemperatur eine Wachstums-abhängige Kohlenstoff-Fütterung gewährleistet. Das erfindungsgemäße Verfahren funktioniert mit einer Fermentation, wobei die Animpfung mit einer Vorkultur erfolgt; es ist aber auch möglich, eine Direktbeimpfung vorzunehmen, was als ein besonderer Vorteil des erfindungsgemäßen Verfahrens anzusehen ist.

Die Ermittlung der obengenannten Parameter erfolgt - wie bereits erwähnt - empirisch. Dabei ist darauf zu achten, daß die Kohlenstoff-Fütterung unter einem Grenzwert bleiben muß, bei dem keine Kohlenstoff-Überfütterung erfolgt. Bei einer Fütterungsrate knapp unter diesem Grenzwert erhält man mittels des obigen Algorithmus die optimale Fütterung in der ersten Wachstumsphase. Nach Erreichen des Grenzwertes wird mit Hilfe des obigen Algorithmus auf eine niedriger liegende weitgehend konstante Pumprate (d.h. ± 10 % des Grundwertes) umgeschaltet. Die Größe der konstanten Pumprate ist ebenfalls empirisch zu ermitteln.

Die Messung der Sauerstofftransferrate wird vorzugsweise vorgenommen, indem mit Standardmeßgeräten der Austrag an Sauerstoff aus dem Fermenter mit dem jeweiligen Eintrag bilanziert wird.

Durch die nachfolgenden Ausführungsbeispiele und durch den Inhalt der Patentansprüche wird die vorliegende Erfindung näher erläutert.

Beispiel 1

Anzuchtbedingungen:

Die Anzucht wird unter folgenden Bedingungen durchgeführt:
Die E. coli Stämme W 3110 M und DH 1 werden in folgendem Medium über Nacht inkubiert

| Hefeextrakt | 0,7 % |
|---|---|
| Bactotryptone | 0,4 % |
| NaCl | 0,4 % |
| Chloramphenicol | 10 μg/ml |

und die Kulturen anschließend mit Glycerin (Endkonz. 15 %) versetzt.

Aus den Suspensionen werden Agarplatten des gleichen Mediums angelegt, 24 Stunden bei 28°C inkubiert und die Vorkulturen (VK) mit einer Einzelkolonie inokuliert.

| VK-Medium: | Tryptone | 2,0 % |
|---|---|---|
| | Hefeextrakt | 1,0 % |
| | NaCl | 0,5 % |
| | Chloramphenicol | 10 $\mu$g/ml |
| pH = 7,2 | | |

100 ml dieser Nährlösungen in 300 ml Erlenmeyerkolben werden nach dem Animpfen für 16 bis 24 Stunden bei 28°C und 220 Upm inkubiert. Die Kultur zeigt dann eine $OD_{578nm}$ von 6.0 bis 8.0.

Aus diesen VK werden die folgenden Hauptkulturen (HK) mit 5 % (bezogen auf VK mit $OD_{578nm}$ = 3,0) beimpft:

HK:  40,0 g/l Hefeextrakt (Oxoid)

1,2 g/l $NaH_2PO_4$ x $H_2O$

8,5 g/l $Na_2HPO_4$ x $2H_2O$

1,0 g/l KCl

2,0 g/l $MgSO_4$ x $7H_2O$

1,0 g/l Zitronensäure

5,0 g/l $NH_4Cl$

4,0 ml/l Spurenelementlösung

0,25 g/l $CoCl_2$ * 6 $H_2O$

0,01 g/l $NiCl_2$ * 6 $H_2O$

0,01 g/l $CuCl_2$ * 2 $H_2O$

0,1 g/l $ZnCl_2$

0,5 g/l $H_3BO_3$

0,3 g/l $Na_2MoO_4$ * 2 $H_2O$

0,1 g/l $NaSeO_3$ * 3 $H_2O$

0,2 g/l $SeSO_4$ * 7 $H_2O$

0,58 g/l $MgSO_4$ * 7 $H_2O$

0,05 g/l EDTA

mit HCl auf pH 2 bis 3 eingestellt)

0,005 g/l Thiamin → sterilfiltriert (5 mg/10 ml → 0.5/50 ml NI)

®Desmophen (Polyole, Bayer AG, Leverkusen)

pH = 6,5

Eine Direktbeimpfung des Fermenters mit nur einer Kolonie ist ohne Aktivitätsverlust ebenfalls möglich.

| Ferm.beding.: | Temp.: | 28°C (Ausnahme siehe Temperatur-Versuch) |
|---|---|---|
| | Vol.: | 3,5 l |
| | vvm: | 0,75 konstant |
| | Upm: | 500 bis 1200 (r = 7 cm) |
| | pH: | 7,0 ± 0,2 (mit $NH_4OH$ 25 %ig/$H_3PO_4$ 1M konstant halten) |
| Fedbatch: | Glycerinlösung: | 525 g Glycerin (99 %)/l HK-Medium (ohne $NH_4Cl$; $MgSO_4$ x $7H_2O$ separat autoklaviert) |

Die Sauerstofftransferrate (OUR) stellt einen geeigneten wachstumsgekoppelten Fermentationsparameter dar, der sowohl der Biomasse, als auch dem physiologischen Zustand der Kultur proportional ist.

Verwendet man den oben angegebenen Algorithmus mit Begrenzung der OUR auf 120 mMol/l*h (empirisch ermittelt), so erzielt man eine optimal angepaßte Glycerinfütterung an die erste Wachstumsphase. Nach Erreichen des Sollwertes schaltet die Pumpe vorschriftsmäßig ab und die OUR fällt.

Nach Erreichen des Sollwertes schaltet man auf eine konstante Pumprate um - dies wird durch obigen Algorithmus veranlaßt - und es wird ein optimales Wachstum erzielt. Für den Fall, daß die maximale Sauerstofftransferrate nicht erreicht wird, wird durch den genannten Algorithmus nach Durchlaufen eines Maximums der Sauerstofftransferrate auf die konstante Pumprate umgeschaltet.

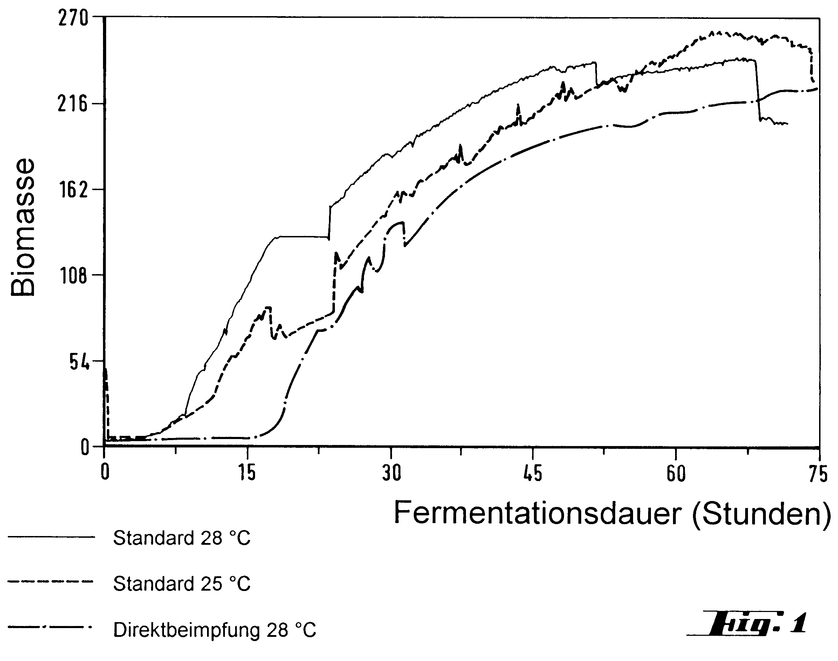

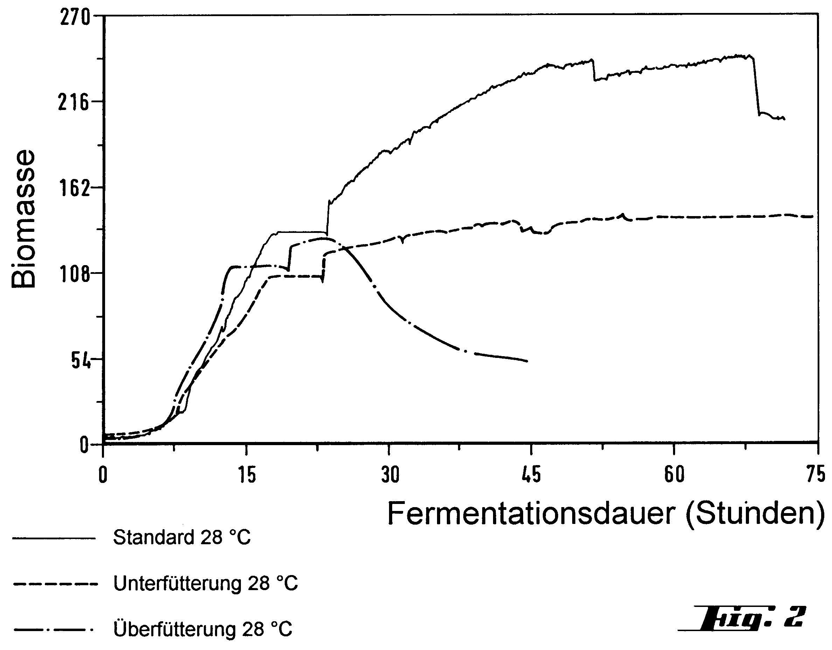

Die Ergebnisse, die in Fig. 1, Fig. 2 und Tabelle 1 wiedergegeben sind zeigen, daß mit Hilfe des erfindungsgemäßen Verfahrens die Fermentation automatisch optimal abläuft, auch wenn die Bedingungen (hier Fermentationstemperatur, Direktbeimpfung, Unterfütterung) geändert werden. Lediglich bei Überfütte-

EP 0 652 288 A1

rung - was nicht der Vorgehensweise gemäß der vorliegenden Erfindung entspricht - bricht die Biomasseproduktion zusammen. Tabelle 2 zeigt, daß auch mit dem Stamm DH 1 eine gute Biomasseproduktion bei geringer Acetatbildung erreicht werden kann.

Tabelle 1

| Organismus W 3110 M: | | | |
|---|---|---|---|
| Bedingungen | Biomasse (g/l) | Glycerinakkumulation (mM/l) | Acetat (mM/l) |
| - Standard | 231,0 | 0 | 4,7 |
| - Unterfütterung | 139,1 | 0 | 5,8 |
| - Überfütterung | 56,4 | 674,0 | 491,0 |
| - 25°C | 227,0 | 0 | 4,7 |
| - 33°C | 169,0 | 0,5 | 7,5 |
| - Direktbeimpfung | 196,0 | 0,8 | 6,2 |

Tabelle 2

| verschiedene Rezipientenstämme von E. coli | | | |
|---|---|---|---|
| Stamm | Biomasse (g/l) | Glycerinakkumulation (mM/l) | Acetat (mM/l) |
| W 3110 M | 231 | 0 | 4,7 |
| DH 1 | 175 | 0 | 5,2 |

**Patentansprüche**

1. Fermentation von sauerstoffabhängigen Mikroorganismen, dadurch gekennzeichnet, daß die Kohlenstoff-Fütterung des Mikroorganismus in der Fermentationsbrühe in Abhängigkeit von der Sauerstofftransferrate erfolgt.

2. Fermentation gemäß Anspruch 1, dadurch gekennzeichnet, daß der Mikroorganismus gentechnisch verändert ist.

3. Fermentation gemäß den Ansprüchen 1 oder 2, dadurch gekennzeichnet, daß der Mikroorganismus zur Art Escherichia coli gehört.

4. Fermentation gemäß einem oder mehreren der Ansprüche 1 bis 3, dadurch gekennzeichnet, daß der Mikroorganismus der Stamm W 3110 M oder DH 1 ist.

5. Fermentation gemäß einem oder mehreren der Ansprüche 1 bis 4, dadurch gekennzeichnet, daß Glutarylacylase produziert wird.

6. Fermentation gemäß einem oder mehreren der Ansprüche 1 bis 5, dadurch gekennzeichnet, daß eine Beimpfung mittels einer Vorkultur erfolgt.

7. Fermentation gemäß einem oder mehreren der Ansprüche 1 bis 5, dadurch gekennzeichnet, daß eine Direktbeimpfung vorgenommen wird.

8. Fermentation gemäß einem oder mehreren der Ansprüche 1 bis 7, dadurch gekennzeichnet, daß die Fütterung in Abhängigkeit von der Sauerstofftransferrate computergesteuert erfolgt.

9. Fermentation gemäß einem oder mehreren der Ansprüche 1 bis 8, dadurch gekennzeichnet, daß die Fütterung des Mikroorganismus mit Glycerin erfolgt.

**10.** Fermentation gemäß einem oder mehreren der Ansprüche 1 bis 9, dadurch gekennzeichnet, daß zunächst die Fütterung des Mikroorganismus proportional zur Sauerstofftransferrote erfolgt bis ein empirisch zu ermittelnder Grenzwert erreicht wird und anschließend eine weitgehend konstante Fütterung erfolgt.

Fig. 1

Standard 28 °C

Standard 25 °C

Direktbeimpfung 28 °C

EP 0 652 288 A1

*Fig. 2*

Standard 28 °C

Unterfütterung 28 °C

Überfütterung 28 °C

Europäisches Patentamt

# EUROPÄISCHER RECHERCHENBERICHT

Nummer der Anmeldung

## EINSCHLÄGIGE DOKUMENTE

EP 94117180.3

| Kategorie | Kennzeichnung des Dokuments mit Angabe, soweit erforderlich, der maßgeblichen Teile | Betrifft Anspruch | KLASSIFIKATION DER ANMELDUNG (Int Cl 6) |
|---|---|---|---|
| X | <u>DE - A - 1 953 430</u><br>(TANABE SEIYAKU)<br>  * Ansprüche 1,4 *<br>-- | 1 | C 12 P  1/00<br>C 12 N  1/00<br>C 12 M  1/36<br>C 12 N  9/80 |
| X | <u>DD - A - 297 444</u><br>(JENAPHARM)<br>  * Anspruch 1 *<br>-- | 1 | C 12 N 15/55<br>//(C 12 P  1/00<br>C 12 R  1:19) |
| X | <u>DD - A - 159 011</u><br>(ADW DER DDR)<br>  * Ansprüche 1,4 *<br>-- | 1 | |
| A | <u>EP - A - 0 196 061</u><br>(HITACHI)<br>  * Ansprüche 1,3; Fig. 5 *<br>-- | 1,8 | |
| A | <u>EP - A - 0 144 474</u><br>(FABRIQUES DE TABAC REUNIES)<br>  * Anspruch 1 *<br>-- | 1 | |
| D,A | <u>EP - A - 0 504 798</u><br>(HOECHST AG)<br>  * Anspruch 1; Beispiel 5 *<br>---- | 1-5 | RECHERCHIERTE SACHGEBIETE (Int. Cl 6)<br><br>C 12 P<br>C 12 N<br>C 12 M |

Der vorliegende Recherchenbericht wurde für alle Patentansprüche erstellt.

| Recherchenort | Abschlußdatum der Recherche | Prüfer |
|---|---|---|
| WIEN | 27-12-1994 | WOLF |